(19) Europäisches Patentamt
European Patent Office
Office européen des brevets

(11) **EP 2 128 598 B1**

(12) **EUROPEAN PATENT SPECIFICATION**

(45) Date of publication and mention
of the grant of the patent:
**23.08.2017 Bulletin 2017/34**

(51) Int Cl.:
*G01N 21/64* *(2006.01)*   *G01N 21/77* *(2006.01)*
*B82Y 15/00* *(2011.01)*   *B82Y 30/00* *(2011.01)*
*G01N 33/543* *(2006.01)*   *G01N 21/552* *(2014.01)*
*G01N 21/25* *(2006.01)*

(21) Application number: **08009827.0**

(22) Date of filing: **29.05.2008**

(54) **Method for detecting analytes or other stimuli by stimuli-responsive polymer brushes**

Verfahren zum Nachweis von Analyten oder anderen Stimuli auf der Basis stimulusempfindlicher Polymerbürsten

Procédée de détection d'analytes ou de stimuli basé sur des brosses de polymère sensibles aux stimuli

(84) Designated Contracting States:
AT BE BG CH CY CZ DE DK EE ES FI FR GB GR HR HU IE IS IT LI LT LU LV MC MT NL NO PL PT RO SE SI SK TR

(43) Date of publication of application:
**02.12.2009 Bulletin 2009/49**

(73) Proprietor: **Leibniz-Institut für Polymerforschung Dresden e.V.**
**01069 Dresden (DE)**

(72) Inventors:
• **Gupta, Smrati**
**01307 Dresden (DE)**
• **Agrawal, Mukesh**
**01307 Dresden (DE)**
• **Uhlmann, Petra,**
**01307 Dresden (DE)**
• **Stamm, Manfred,**
**01705 Freital-Pesterwitz (DE)**

(74) Representative: **Andrae | Westendorp Patentanwälte Partnerschaft**
**Uhlandstraße 2**
**80336 München (DE)**

(56) References cited:
WO-A-03/079402

• **SHANTANG LIU ET AL: "Evaporation-induced self-assembly of gold nanoparticles into a highly organized two-dimensional array" PHYSICAL CHEMISTRY CHEMICAL PHYSICS R. SOC. CHEM UK, vol. 4, no. 24, 15 December 2002 (2002-12-15), pages 6059-6062, XP002500932 ISSN: 1463-9076**
• **TOKAREVA I ET AL: "Nanosensors based on responsive polymer brushes and gold nanoparticle enhanced transmission surface plasmon resonance spectroscopy", JOURNAL OF THE AMERICAN CHEMICAL SOCIETY, AMERICAN CHEMICAL SOCIETY, vol. 126, no. 49, 15 December 2004 (2004-12-15), pages 15950-15951, XP002615986, ISSN: 0002-7863, DOI: 10.1021/JA044575Y [retrieved on 2004-11-18]**
• **Gregory Kalyuzhny ET AL: "Transmission Surface-Plasmon Resonance (T-SPR) Measurements for Monitoring Adsorption on Ultrathin Gold Island Films", CHEMISTRY - A EUROPEAN JOURNAL., vol. 8, no. 17, 2 September 2002 (2002-09-02), pages 3849-3857, XP055331400, WEINHEIM, DE ISSN: 0947-6539, DOI: 10.1002/1521-3765(20020902)8:17<3849::AID-CHEM3849>3.0.CO;2-1**

EP 2 128 598 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

The invention relates to a method for detecting an analyte or another stimulus using a sensor.

There has been an immense interest surrounding the synthesis, physical characterization, and manipulation of nanometer-scale particles in the last decade. Metal/metal oxide nanoparticles have been synthesized out of a variety of materials in very well-defined sizes and a variety of properties, e.g. metallic, conductive, semiconductive, insulating or magnetic nanoparticles. They have been potentially used in a wide spectrum of applications that range from optoelectronic devices to biological sensors. Recently, Au nanoparticles have become an increasingly important material for cutting-edge applications in fabrication of advanced optoelectronic devices by virtue of their tunable optical properties, which are strongly dependent on the particle size and shape, interparticle distance, nature of the protecting organic shell and surrounding media. They are very sensitive to change in surrounding media which can affect their optical parameters. Exploitation of this unusual phenomenon has given rise to new analytical and sensing techniques. Colloidal metal nanoparticles have received much attention in this regard. The colloidal dispersions of Au nanoparticles exhibit particular colors due to collective oscillations of electrons in the vicinity of the surface by visible light and show a colorimetric response to a change in the interparticle distance. For example Nath et al., Anal. Chem. 2004, 76, 5370 - 5378 reported the fabrication of a biosensor made of Au nanoparticles deposited on a glass substrate. Mirkin and co-workers, Nature 1996, 382, 607, have used DNA-tethered colloidal Au particles to develop an optical method for detection of DNA hybridisation. Tokareva et al, J. Am. Chem. Soc. 2004, 126, 15950 - 15951, reported fabrication of nanosensors based on the change in surface plasmon resonance of Au nanoparticles with change in pH of surrounding water. In addition, Emory and Nie, Science, 1997, 275, 1102 - 1106, have studied surface-enhanced Raman scattering (SERS) from single, surface confined Au and Ag nanoparticles. Similarly, the heightened interparticle electric field in deliberately prepared metal nanoparticle-analyte-metal nanoparticle sandwiches has been used to generate strong SERS signals from the sandwiched analyte (Brandt, E.S., Cotton, T.M. Surface-enhanced Raman Scattering. In "Investigations of Surfaces and Interfaces Part B"; Rossiter, B.W., Ed.; John Wiley & Sons: New York, 1993; Vol. IXB, p. 633).

In all these examples, potential applications require positioning of nanoparticles in complex two- and even three-dimensional structures. A crucial issue is the positional stability of particles on a surface, or in a structure, after they have been deposited. A number of protocols ranging from self-assembly to nano manipulation with a scanning probe microscope (SPM) have been proposed to achieve this goal. Among all, two approaches have been most commonly used for particle stabilization.

One involves the use of self-assembled monolayers (SAMs) to cover the particles. SAMs have attracted considerable interest during the last 2 decades and are formed spontaneously by immersing substrates in dilute solutions of amphiphilic molecules in appropriate solvents. A variety of film/substrate systems are known to lead to the formation of these highly ordered monolayer films. Among these systems, alkylsiloxane SAMs on hydroxylated surfaces have proven to be particularly versatile. The alternative approach involves exploitation of polymer chains, tethered by one end to the underlying substrate in brush conformation. This methodology has a number of advantages over the conventional methods of the nanoparticles' stabilization on macroscopic surfaces. For example, polymer brushes can be grown on a variety of functional surfaces exploiting "grafting to" or "grafting from" approaches. Grafted polymer chains can withstand harsh operational conditions of solvent and temperature. Length and grafting density of the polymer brushes can be manipulated in a wide range via selecting polymer chains of suitable molecular weight and grafting such polymers to a substrate surface and via control of reaction conditions when using a grafting from approach wherein the polymers are obtained by direct polymerization on the substrate surface. The grafting density determines the conformation of polymer chains, which in turn, governs the effectiveness of polymer layer for a given application and, above all, polymer brushes are capable of responding to changes of temperature, solvent polarity, pH, and other stimuli, generally by reversible swelling, which lead to changes in optical properties of adsorbed nanoparticles and open a new avenue for nanosensing. Only very few studies have been reported in literature on immobilization of nanoparticles using this approach.

Tokareva et al (*loc. cit.*) reported the fabrication of novel nanosensors based on the variation in distance between the Au nanoparticles adsorbed on poly(2-vinylidene) polymer brushes and those deposited on the substrate, with the change in pH of surrounding media. The gold nanoparticles are randomly distributed throughout the polymer brush layer and bound to the polymer brush by hydrophobic-hydrophobic interaction. For signal detection is used transmission surface plasmon resonance spectroscopy which requires a complicated set-up. To detect the required signal it is necessary to cover the substrate surface with an extra gold nanoparticle layer.

Mitsuishi et al., Langmuir 2007, 23(14), 7472 - 7474, demonstrated temperature induced change in optical properties of gold nanoparticles, assembled on a substrate with temperature responsive PNIPAM (Poly-N-isopropylacrylamide) brushes.

L. Ionov et al., Adv. Mater. 2006, 18, 1453 - 1457, describe sensors based on fluorescence interference contrast (FLIC) of semiconductor nanoparticles near a reflecting silicon surface. FLIC arises from the interference between light that is directly emitted (or absorbed) by a nanoparticle and the light that is reflected by the mirror surface. The intensity of the detected fluorescence light becomes a periodic function of the nanoparticle distance from the surface. CdSe nanoparticles are randomly distributed in a polymer layer formed from poly(2-vinylpyridine) polymers anchored with one end to the substrate surface. The CdSe nanoparticles are bound to the polymer molecules by hydrophobic-hydrophobic interaction. The polymer layer swells or shrinks as a response to the presence of an analyte thereby influencing the distance between the nanoparticles and a reflecting surface.

In EP 1 215 485 A1 are described selective chemical sensors based on interlinked nanoparticle assemblies. The sensor comprises a nanoparticle film comprising a nanoparticle network formed of nanoparticles interlinked by linker molecules. The linker molecules have at least two linker units that can bind to the surface of the nanoparticles. By introducing selectivity enhancing units in the linker molecule, the selectivity of the nanoparticle film towards target analytes can be enhanced. A fine tuning of the selectivity can be achieved by including a fine-tuning unit in the vicinity of the selectivity-enhancing unit.

US 2005/019849 A discloses a polymer layer for a sensor, wherein the polymer layer has nanoparticles embedded therein which impart to the polymer layer recognizing properties as well as transducer properties.

In US 2006/0050268 A1 is disclosed a surface-enhanced Raman Spectroscopy (SERS) system, comprising:

- one or more nanosensors, each nanosensor further comprising at least one Raman-active molecule coupled to a metal nanoparticle;

- an electromagnetic radiation source;

- means configured to direct the electromagnetic radiation source onto disposed said one or more nanosensors and additionally configured to collect a SERS spectra resulting from said directed electromagnetic radiation source;

- two or more optical detectors configured to monitor radiometric signals of said SERS spectra; and

- means to determine the presence and concentration of one or more target molecules, and/or changing physical and/or chemical conditions of an analyte resulting from the monitored radiometric signals.

In US 2006/0286684 A1 is disclosed a sensor comprising silver nanoparticles. The nanoparticles are formed in the presence of a polymeric stabilizer. The silver nanoparticles are capable for interaction with an analyte or receptor which in turn is capable of binding an analyte.

WO 03/079402 A2 relates to an adsorbent chip, which includes three components, a substrate, an intermediate layer of linker arms and an adsorbent film, which is attached to the linker arms, and methods for using the chips to perform assays.

S. Liu et al., Phys. Chem. Chem. Phys. R. Soc. Chem. UK 2002, 4(24), 6059 - 6062 relates to a method for fabricating a self-organized two-dimensional (2D) array of gold nanoparticles on a silicon substrate.

G. Kalyuzhny et al., Chemistry 2002, 8(17), 3849 - 3857 relates to Transmission surface-plasmon resonance (T-SPR) measurements for monitoring chemical or physical adsorption on ultrathin gold island films.

Although the results obtained with the above-described sensors are encouraging, there still is a need for new sensor types based on nanoparticles which have a high sensitivity, a high stability in performance and are easy to use.

It therefore is an object of the invention to provide a method for detecting an analyte or another stimulus which uses a new sensor type, which may be obtained easily, has a high stability against the environment and is easy to operate.

To solve this object, the present invention provides a method according to claim 1. Preferred embodiments of the method are subject of the depending claims.

The sensor employed in the method uses a polymer brush with nanoparticles bound to the free ends of the brush. The polymer brush may interact with an analyte present or may respond to a stimulus, e.g. temperature, which in turn induces a swelling of the polymer layer. Since the nanoparticles are bound to the free ends of the polymer brush, a swelling of the polymer layer influences the interparticle distance of the nanoparticles. A physical parameter, that is influenced by the interparticle distance of the nanoparticles may then be used to detect the presence of the analyte or the stimulus.

In the sensor employed in the method according to the invention, the nanoparticles are not randomly distributed within a polymer layer fixed to a substrate surface but are fixed to a defined position at a terminal end of a polymer brush. The terminal end of a polymer brush is to be understood as an end that is the farthest away from the end of the polymer anchored to the substrate following the contour of the polymer molecule. In a linear polymer only two ends are present, one forming the anchor to the substrate and the other being designated a terminal end is free floating. In a branched polymer a terminal end is to be understood that end of the polymer, that is, following the contour of the polymer, the farthest away from the end forming the anchor to the substrate, i.e. is the other end of the main chain of the polymer.

The position of the nanoparticles within a sensing portion of the sensor formed by the polymer brush and the nanoparticles therefore is well defined. The nanoparticles basically form a nanoparticle layer covering a surface of the polymer brush and features of such nanoparticle layer may be used to sense an analyte.

For example, such nanoparticle layer has a quite ordered structure in which the nanoparticles are arranged in closed relationship. Therefore e.g. the interparticle distance between two nanoparticles may be used as an analyzing tool to analyze a feature of an analyte applied to the sensor.

Besides the nanoparticles arranged at the free ends of the polymer brush additional nanoparticles may be positioned inside the polymer brush. However, preferably most of the nanoparticles bound to the polymer brush are arranged at the free ends of the polymer brush. Preferably, more than 50 %, more preferred at least 70 %, particularly preferred at least 90 % of the nanoparticles are provided at the free ends of the polymer brush.

The amount of polymer grafted to nano-particles may be determined e.g. by Attenuated Total Internal Reflection Fourier Transform Infrared Spectroscopy (ATR-FTIR) which can be used to generate spectra of monolayers, polymer brushes and nano-particles having specific IR-sensitive functional groups. With the ATR-FTIR spectroscopy, the infrared beam analyses a surface by internal reflection of the light, i.e. a beam of radiation enters from a more optical dense medium e.g. an ATR crystal with high reflective index into a less optical dense medium e.g. a sample.

The matter of interest, e.g. a linear polymer molecule, branched molecule, nano-particles or their mixtures, is positioned on the crystal surface and the infrared light penetrates the surface via an evanescent field. This penetration is typically at a depth of few micrometers.

The amount of polymer grafted to the nano-particles can be determined from a calibration curve Y/X, wherein Y is the FTIR Integral absorbance band of a functional group reacting with a nano-particle, e.g. a carboxylic group or an amide group, and X is the amount of polymer contained in the mixture of polymer and nanoparticle, e.g. in wt.% or mg. The position of the nanoparticles in the polymer brush may be influenced by the synthesis conditions used to fix the nanoparticles to the polymer brush.

Besides a well ordered layer formed of nanoparticles, the sensor comprises a polymer brush. Such brush is formed by polymer chains tethered with one end to a substrate surface. Preferably such polymers form linear chains, but branched polymer chains may also be used for some applications. The polymers may be present in a well ordered structure with all polymer chains being directed away from the substrate surface in an expanded, stretched conformation. However, the polymer brush may also be present in a collapsed form with the polymer chains being more in an irregular or coiled conformation and taking e.g. the form of a clew. With the nanoparticles being bound to the terminal end of the polymer brush, the interparticle distance of the nanoparticles therefore is influenced by the conformation of the polymer brush.

The polymer brush now may be used to analyze a stimulus applied to the sensor, e.g. temperature or pH, which influences the conformation of the polymer chains thereby influencing the structure or regularity of the layer formed by nanoparticles bound to terminal ends of the polymer brush. A physical parameter influenced by the interparticle distance between two nanoparticles may then be used to detect the influence of the stimulus on the conformation of the polymer brush.

The polymer brush may also act as a sensor medium for an analyte to be detected. Depending on the nature of the analyte, the polymer brush may swell by interacting with analyte molecules being located between individual polymer molecules of the brush, or may collapse forming a layer of irregular structure in case the analyte is not interacting with the polymer brush. The sensor may therefore be also used to detect the polarity of an analyte, e.g. of a solvent.

For generating a detectable signal, it is sufficient to provide a layer of nanoparticles which are held in position by the polymer brush. In the sensor employed in the method according to the invention, it is not necessary to provide e.g. a reflecting layer on the substrate surface to use e.g. surface plasmon resonance or FLIC as an analyzing tool. Instead it is possible to use quite simple methods, e.g. UV-VIS-spectroscopy, to analyze the presence of an analyte at any substrate on which polymer brushes can be prepared. However, a sensor may be modified in such way, that it is possible to also use techniques such as e.g. surface plasmon resonance or FLIC for signal detection.

According to the invention therefore is provided a method for detecting an analyte or another stimulus comprising the steps:

- providing a sensor comprising:

  - a substrate, wherein the substrate does not carry a gold layer;

  - a polymer brush formed of polymer molecules anchored by a first end to a surface of the substrate and having a second, free end located on a terminal end of the polymer molecule opposite to the first end;

  - nanoparticles fixed to the free second end of the polymer brush,

- selecting a physical parameter of the sensor;

- determining the physical parameter thereby obtaining a reference value;

- applying a stimulus to the sensor;

- determining the physical parameter of the sensor in the presence of the stimulus, thereby obtaining a measured value;

- determining a difference between the reference value and the measured value,

wherein UV/VIS-absorption of the sensor is used to determine the presence of an analyte or another stimulus.

In the sensor employed in the method according to the invention, the nanoparticles are fixed to the ends of a polymer brush and therefore are at a defined position within the sensor and not randomly distributed within a polymer layer as in sensors of similar type described in the prior art. The nanoparticles may form a layer covering the polymer brush wherein the nanoparticles are in close relationship to each other.

The nanoparticles are bound to the ends of the polymer molecules forming the polymer brush. The polymer molecules are tethered with one end to the substrate surface but retain flexibility. They may take a more linear shape when being in a stretched conformation or may take a more dense shape when being in a collapsed conformation. The conformation of the polymer chain may be influenced by physical parameters, e.g. temperature, pH, ionic strength, or may be influenced by the presence of an analyte. E.g. an analyte having a polarity similar to the polarity of the polymer molecules will interact with the polymer brush. The adsorption of the analyte will induce a swelling of the polymer brush with the polymer molecules taking more of a stretched expanded conformation. When taking an analyte that has a polarity significantly different from the polarity of the polymer molecules, then the analyte will only weakly interact with the polymer brush. The polymer brush will collapse with the polymer molecules taking a more dense, cluster-like conformation.

Since the nanoparticles are bound to the free ends of the polymer brush, the conformation of the polymer chain will influence e.g. the interparticle distance between nanoparticles, in particular the distance between the nanoparticles arranged at the free ends of the polymer brush. Therefore, every parameter being influenced by the interparticle distance between the nanoparticles may be used as a tool to detect the analyte.

The sensor basically comprises a substrate, a polymer brush and nanoparticles.

There are no particular restrictions to the substrate other than that it does not carry a gold layer. The substrate may play a neutral role and may act only as a basis for anchoring the polymer brush in a defined position. However, the substrate may also take an active position in the sensing mechanism of the sensor and may e.g. act as an electrode to detect a signal generated from the sensor. According to the present invention, an analytic technique is used that does not need the substrate surface to induce a signal but e.g. only uses the relative position of the nanoparticles to generate a signal. The method used is UV-VIS. Such method will be discussed further below.

The substrate preferably should have a regular planar surface such that a regular and defined structure of the polymer brush may be obtained. Suitable substrates are e.g. a glass /quartz plate or a silicon wafer. However, other materials may also be used. The substrate may be stiff or may be flexible and take e.g. the form of a thin foil. Such foil may be made e.g. from a metal or a plastic material, i.e. an organic polymer. The substrate may be formed homogeneously of a particular material. However, it is also possible to use a substrate comprising several layers made of different materials. An example for such a substrate is e.g. a silicon wafer, on top of which is provided a layer of silica. Such silica layer may act as adhesion enhancing layer to strengthen positional stability of the polymer brush on the substrate. Further, the substrate may comprise domains made of different materials and may e.g. be formed by a microchip comprising electrical circuits to detect a signal generated within the sensor.

On the surface of the substrate is provided a polymer brush formed of individual polymer molecules tethered with one end to the substrate surface. Every type of bond is suitable to anchor the polymer molecules to the substrate as long as the bond provides sufficient strength to withstand the conditions under which the sensor will be used to sense a physical stimulus, e.g. the presence of an analyte. The linkage between the polymer and the substrate may be e.g. by a coordinative bond or by a covalent bond.

To the terminal end of the polymer molecule opposite to the end tethered to the substrate is bound a nanoparticle. As already explained above, the terminal end of the polymer molecule is understood to be that end of the polymer molecule that, following the contour of the polymer molecule, is the farthest away from the end of the polymer molecule bound to the substrate surface.

Every type of bond may be used to bind the nanoparticle to the terminal end of the polymer molecule. The bond between the nanoparticle and the polymer molecule should be of sufficient strength such that the bond can withstand conditions under which the sensor is used.

A single nanoparticle may be bound to an individual polymer molecule. However, to achieve a firm fixation of the nanoparticle to the polymer brush, it is preferred that several polymer molecules bind to an individual nanoparticle.

The nanoparticles bound to the free ends of the polymer molecules of the polymer brush approximately form a layer of densely packed particles. Preferably they occupy at least 3 % of the area provided on the substrate surface by the polymer brush, more preferred more than 10 %. Although densely packed, the nanoparticles preferably do not occupy the complete surface area provided by the polymer brush. Preferably neighbored nanoparticles do not touch each other, i.e. single nanoparticle adsorption to the polymer brush is preferred. A single nanoparticle adsorption may be confirmed e.g. in the case of gold nanoparticles by the absence of the optical signature of aggregate formation in the adsorption spectrum of Au nanoparticles.

According to an embodiment up to 30 %, preferably up to 20 %, more preferred up to 15 % of the surface area provided by the polymer brush is occupied by nanoparticles. To obtain a sufficient response to the presence of a stimulus and to obtain a signal of sufficient strength it is preferred, that at least 4 % of the surface area provided by the polymer brush, more preferred at least 5 % of the surface area provided by the polymer brush is occupied by nanoparticles. The surface area provided by the polymer brush corresponds to the surface on the substrate occupied by the polymer brush. The surface area may be determined e.g. on a photograph obtained by suitable electron microscope techniques, e.g. atomic force microscopy.

The polymer molecules forming the polymer brush may have any structure and may be branched or linear. Linear polymer molecules are preferred since they allow an easy swelling and shrinking of the polymer brush as a response to the presence of an analyte or other stimulus.

The individual polymers of the polymer brush may be cross-linked. However, since cross-linking renders swelling of the polymer brush in the presence of an analyte or another stimulus more difficult, it is preferred that the individual polymer molecules of the polymer brush are not cross-linked.

The chain length of the polymer molecule may be selected within a broad range and is also dependent on the nature of the repeating units of the polymer molecule. The polymer molecule should have a length sufficient that the polymer brush may swell, shrink, or collapse as a response to the presence or absence of an analyte or another physical stimulus. However, the length of the polymer molecule should not be selected too long since then the response of the sensor to a variation in e.g. an analyte concentration may be too slow for practical applications. The length and the chemical nature of the polymer molecules therefore is selected according to the analytical problem to be solved.

The nanoparticles may be made of every suitable material and may be formed e.g. of a metal, a semiconductor, or even may be a magnetic nanoparticle. For some application it may also be suitable to use an isolating material for the nanoparticles, e.g. a metal oxide or a material that has photo luminescent properties or fluorescent properties. Nanoparticles as used in the sensor employed in the method according to the invention are particles preferably having a size of less than 150 nm, more preferred less than 100 nm and particularly preferred have a size selected within a range of 1 to 50 nm. According to a further preferred embodiment, the nanoparticles have a diameter of less than 20 nm, particularly preferred 2 to 20 nm. Preferably the nanoparticles have a narrow size distribution. Preferably, more than 80 % of the nanoparticles have a size within a range of $\pm$ 10 % of the mean size of the nanoparticles. The size of the nanoparticles may be determined e.g. by TEM (Transmission electron microscopy), DLS (Dynamic Light scattering), SEM (scanning electron microscopy) or AFM (Atomic Force Microscopy).

The thickness of the polymer brush in a dry state and the average diameter of the nanoparticles are preferably selected such that the thickness of the polymer brush in a dry state is at least 80 % of the average nanoparticle diameter. According to an embodiment, the thickness of the polymer brush in a dry state is less than 500 % of the average nanoparticle diameter. When the thickness of the polymer brush is selected too large, e.g. diffusion of an analyte into the polymer brush may become slow and the response of the sensor towards a stimulus is retarded. Preferably the thickness of the polymer brush in a dry state is selected in a range of 100 to 150 % of the average nanoparticle diameter.

The nanoparticles preferably have a spherical shape but may also take the shape of a small platelet or a rod. The nanoparticle may be massive or may be hollow. An example for a hollow nanoparticle is e.g. a fullerene or a carbon nanotube. Filled nanoparticles are preferred.

As already discussed above, the linkage between the nanoparticle and the polymer molecule should be of sufficient strength such that the sensor may also withstand harsh conditions during use. This will enable the sensor to be used over a prolonged time e.g. in a routine application. According to a preferred embodiment, the polymer brush comprises terminal bonding groups at the second, free end. A bonding group shall be every group that may form a linkage between the free end of the polymer and a nanoparticle. The linkage may be formed by every type of bond. The bond between the polymer molecule and the nanoparticle may be a coordinative bond or a covalent bond. However it is also possible to achieve a bond by ionic interaction, hydrogen bonding or van-der-Waals forces acting between a terminal bonding group positioned at the terminal end of the polymer molecule and a nanoparticle. The terminal bonding group positioned at the terminal end of the polymer molecule may bind directly to the nano-particle surface. For example, it is known that thiol groups bind quite firmly to a surface of a gold particle. However, it is also possible to modify the surface of the nanoparticle by suitable groups which then will form a bond to the terminal bonding group of the polymer molecule. Such type of interlinking between the bonding group of the polymer molecule and the nano-particle may e.g. be formed by a ionic bond or a hydrogen bond.

In such embodiment an anionic group may be positioned as a terminal bonding group at the terminal end of the polymer molecule and cationic groups may be positioned on the surface of the nanoparticle such that the nanoparticle may be bound to the terminal end of the polymer molecule by ionic bonding. However, it is also possible to provide a cationic group as a bonding group at the terminal end of the polymer molecule and anionic groups on the surface of the nanoparticle.

According to a preferred embodiment, the bonding group located at the free terminal end of the polymer molecule is selected from the group of hydroxyl group, amino group, carboxyl group, thiol group, amide group and ester group.

It has been found, that the nanoparticles bound to the free ends of a polymer brush have high positional stability when bound to the free terminal ends of the polymer molecules by hydrogen bonding, ionic bonding or covalent bonding. Preferably, the nanoparticles are bound to the polymer brush by hydrogen bonding.

Preferably, the polymer molecules forming the polymer brush only carry a bonding group at their terminal end and that terminal bonding group may bind to a nanoparticle. However, for some applications it may be suitable, that besides the bond formed at the terminal end of the polymer molecule further binding sites for binding nanoparticles are provided in the chain or a branch of the polymer molecule.

It has already been explained that the polymer brush may basically be formed from a polymer of any structure as long as e.g. the polymer brush may respond to the presence of an analyte or another stimulus by swelling or collapsing. To achieve a high sensitivity, however, it is preferred that the polymer brush is formed of linear polymer molecules. A linear polymer molecule is a polymer that has no branches. This provides a high flexibility to the polymer chain and a high sensitivity to swelling while interacting with an analyte in surrounding media.

The structure of the polymer molecules forming the polymer brush is selected according to the chemical nature of the analyte to be detected, and can be polar, non-polar or can contain a combination of functional groups. The polymer may comprise polar groups to detect the presence of a polar analyte. Such polymer molecule may be formed e.g. by a polyglycol, like polyethylene glycol or polypropylene glycol. Other exemplary groups that may influence the polarity of the polymer molecule are e.g. halogen groups, like a chloride group, and ionic groups, e.g. anionic groups such as a sulfonate group or a carboxy group or cationic groups such as quaternary ammonium groups e.g. a pyridinium group. Amongst various organic materials, weak polyelectrolytes have been found as most suitable for pH sensing in aqueous medium. Suitable weak polyelectrolytes containing carboxylic groups (polyacids) are e.g. polyacrylic acid and polymethacrylic acid. Suitable weak polyelectrolytes contaning quaternary ammonium groups (polybase) are e.g. poly-2-vinylpyridine and poly-4-vinylpyridine. Such polymers allow e.g. manufacturing of a pH-sensitive sensor since the pH of a solution determines the charge of the pyridine or carboxylic group contained in the polymer brush. A sulfonate polymer, e.g. poly(styrene sulfonate), may be used e.g. to detect the ionic strength of a solution.

Preferably, however, the polymer molecule is formed by a molecule that is basically non-polar. The sensor will then be most sensitive to molecules having a non-polar nature or comprising large non-polar groups. According to an embodiment, the polymer brush is formed from hydrocarbon repeating units. Such hydrocarbon repeating units preferably comprise only carbon and hydrogen.

The polymer molecule may be a saturated hydrocarbon molecule. Examples for such saturated hydrocarbon molecules are polyethylene or polypropylene.

However, the hydrocarbon polymer may also comprise aromatic groups, like a phenyl group or a benzyl group. Such groups may strengthen the interaction between e.g. a non polar analyte and the polymer brush thereby increasing the sensitivity of the sensor.

Preferred hydrocarbon repeating units for forming the polymer brush are selected from the group of styrene, 2-vinyl pyridine, 4-vinyl pyridine, N-isopropyl acrylamide, ethylene, propylene, (Meth)acrylate, Methyl(meth) acrylate. If not already present, a terminal bonding group may be introduced into the polymer before or after fixing the polymer brush onto the substrate surface.

The polymer brush preferably is of sufficient thickness such that a significant swelling is achieved as a response to the presence of an analyte. The thickness of the layer formed by the polymer brush may be measured e.g. by ellipsometry. In a dry state, i.e. in a state where basically no analyte molecules are present within the polymer brush, the layer thickness of the polymer brush is preferably selected within a range of 3 to 20 nm, more preferred 4 to 10 nm.

To obtain a polymer brush of sufficient thickness it is preferred according to an embodiment, that the polymer brush has a chain length selected within a range of 10 to 5.000 repeating units, preferably 50 to 500 repeating units.

The repeating units preferably are selected to have a molecular weight within a range of 30 to 300 g/mol, more preferred 50 to 200 g/mol.

The grafting density of the polymer molecules should be high enough to achieve a strong swelling indicating the presence of an analyte or other stimulus but should be not too high such that the polymer brush may become too stiff not allowing easy access of analyte molecules. The grafting density of the polymer molecules is also depending on the size of the polymers. The grafting density is calculated on the basis of the molecular weight of the polymer molecules and the polymer layer thickness determined by ellipsometry. To obtain a sufficient response to the presence of an analyte or other physical stimulus, the grafting density of the polymer brush on the substrate is preferably selected within a range of 0.05 to 0.5 $nm^{-2}$, more preferred 0.1 to 0.3 $nm^{-2}$. The distance between grafting points of the polymer molecule on the substrate surface is preferably selected within a range of 1.5 to 10 nm, more preferred between 2 and 5 nm.

To allow use of the sensor over a prolonged time also under harsh conditions, the linkage between polymer molecules and substrate should be of sufficient strength. The polymer molecules may be bound directly to the substrate surface e.g. by forming a covalent bond or a hydrogen bond to a hydroxyl group present on the substrate surface. The stability of the linkage between substrate and polymer brush may be enhanced according to a preferred embodiment by providing an anchoring layer on the substrate to which the polymer molecules are bound. The anchoring layer may form multiple bonds towards the substrate thereby increasing the bonding strength between substrate and anchoring layer. The bonds formed between substrate and anchoring layer may be of any type and may be a covalent bond, a coordinative bond, a ionic bond, a hydrogen bond or non-polar interaction by van-der-Waals forces.

The anchoring layer then may provide binding sites to bind the polymer molecules of the polymer brush to the anchoring layer. Such bond between the anchoring layer and the polymer molecules may be of any nature and may be a covalent bond or a non-covalent bond, e.g. a ionic bond, a hydrogen bond or a non-polar bond. Covalent bonds are preferred.

To further improve the interaction between substrate and anchoring layer, an adhesion enhancing layer may be provided on the substrate and the anchoring layer is then positioned on top of the adhesion enhancing layer. Such an adhesion enhancing layer may e.g. be a thin layer of native silicon oxide grown on the surface of a silicon wafer.

According to an embodiment, the anchoring layer is formed of anchoring groups provided on the substrate surface to bind the polymer brush to the substrate surface. Such groups may be obtained by e.g. oxidizing the surface of a silicon wafer to thereby provide a thin layer of silica.

According to another embodiment, the anchoring layer is provided by reacting a small, at least bifunctional molecule with the substrate. One of the functional groups will then bind to the substrate surface whereas the other functional group may be used to bind one end of the polymer molecules forming the polymer-brush. An exemplary compound that may be used as bifunctional molecule is 3-glycidoxypropyl trimethoxy silane. Other functionalized silanes may be used as well.

According to a further embodiment, the anchoring layer is formed of polymers comprising reactive groups. A reactive group is understood to be a group that has sufficient reactivity to react with a group present on the substrate surface or at the polymer molecule to thereby form a covalent bond. The polymer may then react with groups present on the substrate surface, e.g. a hydroxyl group. Since the polymer will react with several groups present on the substrate surface, the polymer will be bound to the substrate surface quite firmly. After applying the anchoring layer to the substrate surface there are a number of reactive groups still present which may then react with a group present at the polymer molecule to form a preferably covalent bond to the polymer molecule forming the polymer brush. Suitable reactive groups present in the polymer forming the anchoring layer are e.g. epoxy groups, trichlorosilane, ethoxy/methoxy silane.

The material the nanoparticles are made of is selected according to the analyte to be detected and the analyzing method, i.e. the physical parameter used to detect a variation of the sensor effected by the presence of an analyte. The nanoparticles may be made e.g. of an isolating material, e.g. an isolating metal oxide, a semiconductor, or a metal. Preferably, the nanoparticles are made from metal or alloys of metals.

Preferably, the metal is selected from the group of gold, silver, copper, platinum or palladium and their alloys. Those metals are particularly preferred, because an optical method which is UV/VIS, is used as a detection method for detecting the presence of a stimulus. Gold is particularly preferred as a material for the nanoparticles. Other materials may also be used for the nanoparticles. Exemplary semiconducting materials are ZnO, $TiO_2$. Exemplary photoluminescent materials are CdTe, CdSe.

In the sensor employed in the method according to the invention, the nanoparticles are immobilized on the tips of a polymer brush. Immobilization may be achieved by interaction of a terminal bonding group located at the terminal end of a polymer molecule forming the polymer brush and the nanoparticle surface. The terminal bonding group may bind directly to the nanoparticle surface. However, the nanoparticle surface may also be modified in such way to provide functional groups that may bind to the bonding group of the polymer brush. According to an embodiment, the nanoparticles are functionalized by surface molecules comprising a functional group that may bind to the bonding group of the polymer brush.

The functional group of the surface molecule is selected according to the bonding group present at the terminal end of the polymer molecule such that the functional group may interact with the bonding group thereby forming a bond. The bond may be a covalent bond or a non-covalent bond. Suitable non-covalent bonds are e.g. ionic bonds or hydrogen bonds.

According to a preferred embodiment, the functional group is selected from the group of hydroxyl group, amino group, carboxyl group, thiol group, ester group and amide group.

To attach the surface molecule to the nanoparticle surface, the surface molecule may be bifunctional comprising a linking group forming a bond towards the nanoparticle surface and a functional group forming a linkage to the terminal bonding group of the polymer molecule of the polymer brush. The linking group is selected according to the material of the nanoparticle. According to an embodiment, the linking group is selected from sulfur-containing groups and nitrogen containing groups and preferably is a thiol group or an amine group.

The surface molecule preferably is a small molecule of low molecular weight. Preferably, the molecular weight of the surface molecule is selected within a range of 50 to 500 g/mol, more preferred 100 to 350 g/mol.

Suitable surface molecules are e.g. 11-mercaptoundecanoic acid, 3-aminopropyl trimethoxysilane or mercaptoamines.

The sensor employed in the method according to the invention may be easily synthesized by simple steps. Therefore, a process for obtaining a sensor as described above is also disclosed.

The method described herein comprises the steps of:

- Providing a substrate;

- Providing a polymer brush formed of polymer molecules anchored by a first end to a surface of the substrate and having a second, free end located on a terminal end opposite to the first end;

- Fixing nanoparticles to the second end of the polymer brush.

As a first step a substrate is provided. Details of the substrate were already discussed above. Before starting to anchor the polymer brush on the substrate surface, the substrate may be cleaned by known methods and may be surface modified, e.g. by providing a thin oxide layer to improve the linkage between polymer brush and substrate. Further, e.g. a mask may be provided on top of the substrate to cover such parts of the substrate surface that should not be covered by the polymer brush.

To the substrate surface is then anchored a polymer brush by binding polymer molecules with one end to the substrate surface.

Various methods are available for binding the polymer brush to the substrate surface.

According to an embodiment, reactive groups may be provided directly on the substrate surface by e.g. surface oxidation of the substrate surface thereby generating e.g. hydroxyl groups that may be used to link the polymer molecule to the substrate surface. To increase the number of reactive groups present for binding the polymer molecules forming the polymer brush, an anchoring layer may be provided. Such anchoring layer may be obtained by applying a polymer to the substrate surface wherein the polymer comprises reactive groups that may react or interact with groups present on the substrate surface. A suitable exemplary polymer is a polymer comprising epoxy groups which may react with hydroxyl groups present on the substrate surface. An exemplary polymer comprising reactive epoxy groups is poly(glycidyl methacrylate). A portion of the glycidyl groups may react with hydroxyl groups present on the substrate surface, e.g. a silicon wafer provided with a thin layer of silicon oxide. The anchoring layer is firmly bound to the substrate surface with the remaining portion of the glycidyl groups provided for forming a bond to the polymer molecules of the polymer brush.

The polymer molecules may be formed by every suitable method, e.g. a "grafting to" or a "grafting from" method. According to a "grafting to" method, the polymer is provided and grafted to the substrate surface, e.g. via an anchoring layer.

According to a "grafting from" method the polymer is formed from monomers by a graft polymerization directly on the substrate surface. Such method is also known as surface initiated polymerization. The most common way of the "grafting from" method includes at first the modification of a substrate surface to obtain an anchoring layer, usually using bifunctional compounds such as silane compounds e.g. 3-aminopropyl-triethoxysilane. The second step usually is the introduction of an initiator for polymerization onto a modified substrate surface. There many different types of initiators can be used e.g. azo-initiators such as 4,4'-azobis(4-cyanvaleric acid), 4,4'-azobis(4-cyanpentanoic acid), 4,4'-azobis(isobutyronitrile) or photoreactive phenylazido in the case of radical polymerization. In the next step the "grafting from" of a monomer is performed from a solution or a gas phase. Polymer brush density, polymer brush thickness and surface uniformity can be influenced by an appropriate choice of monomer size and chemical composition, monomer concentration, temperature and additional initiator e.g. non attached to the solid substrate surface. By using the "grafting from" approach, it is possible to obtain monolayers of a variety of polymers having a thickness of between a few nanometers and a few micrometers. The properties of the layers can be readily adjusted by choosing an appropriate monomer. "Grafting from" allows a linear scaling between the degree of polymerisation and equilibrium thickness of a film. "Grafting from" also allows preparation and determination of grafted polymers where the average distance between grafting points is much smaller compared to the grafted polymers prepared by a "grafting to" method.

There are several methods to carry out the "grafting from" polymerisation, e.g. free radical polymerisation, cationic polymerisation, ring-opening polymerisation, atom transfer free-radical polymerisation (ATRP), anionic polymerisation, etc. All above mentioned methods are suitable for polymerizing different types of monomers on a variety of substrate surfaces.The polymer molecule bound to the substrate surface preferably is functionalized at its end thereby providing a terminal bonding group for binding the nanoparticles. This may be achieved by selecting an appropriate bifunctionalized polymer molecule that is grafted with one functional group to the substrate surface leaving the other functional group as a bonding group for binding the nanoparticles. According to another embodiment, the polymer is functionalized at its terminal end after grafting the polymer to the substrate surface, e.g. by selective oxidation of the terminal end of the polymer molecule.

The nanoparticles are then bound to the bonding groups of the polymer brush. Suitable nanoparticles were already described above.

The position of the nanoparticles within the polymer brush may be influenced by the reaction conditions used to fix the nanoparticles to the polymer brush. In a case, the nanoparticles should be fixed within the bulk of the polymer brush, the nanoparticles are grown in the presence of the polymer brush. Groups present in the bulk of the polymer brush may then act as crystallization germs such that a nanoparticle is obtained bound to the functional group, e.g. a nitrogen-containing group, like an amide group.

In the sensor employed in the method according to the invention, the nanoparticles are fixed to the tips of the polymer brush. To selectively fix the nanoparticles to the ends of the polymers forming the polymer brush, preferably a dispersion of the nanoparticles is provided and said dispersion is applied to the polymer brush anchored by a first end to the surface of the substrate. The nanoparticles contained in the dispersion will then interact only with e.g. a bonding group provided at the second, free end of the polymer molecules forming the polymer brush whereas, if any, only few nanoparticles will intrude into the bulk of the polymer brush to eventually bind to a group provided in the bulk of the polymer brush.

Preferably, the nanoparticles are modified by surface molecules providing a functional group that may interact with the terminal bonding group of the polymer molecule to form a covalent or noncovalent bond. The nanoparticles may be provided dispersed in a suitable liquid and the substrate carrying the polymer brush is then incubated with the nanoparticle dispersion. The reaction conditions for incubating the nanoparticle dispersion may be selected suitably. Preferably, the substrate with the polymer brush bound to it is first treated with a solvent such that the polymer brush is in a swollen state. The substrate with the swollen polymer brush placed on it is then dipped in a dispersion of the nanoparticles in a similar or the same solvent used for swelling the polymer brush. The substrate with the polymer brush is preferably incubated at room temperature for a prolonged time, preferably more than 4 hours, more preferred 8 to 24 h. Preferably, the dispersion of the nanoparticles is stirred during incubation. The substrate with the immobilized nanoparticles is then taken out of the nanoparticle dispersion and rinsed with sufficient solvent, preferably the same solvent used for binding the nanoparticles to the polymer brush, to remove weakly or unbound nanoparticles. A suitable solvent for immobilization of nanoparticles on a non-polar polymer brush is e.g. toluene, but other solvents may be used as well.

Preferred embodiments of the nanoparticles, polymer brush and substrate were already explained with reference to the sensor employed in the method according to the invention. Such embodiments apply accordingly to the method according to the invention.

The sensor employed in the method according to the invention may be used to detect an analyte in a medium or to detect another stimulus, e.g. temperature, pH or ionic strength of a solution. An object of the invention therefore is directed to a method of detecting an analyte or other stimulus by using the above described sensor.

The method for detecting an analyte according to the invention comprises the steps:

- Providing a sensor as defined in claim 1;

- Selecting a physical parameter of the sensor;

- Determining the physical parameter thereby obtaining a reference value;

- Applying a stimulus to the sensor;

- Determining the physical parameter of the sensor in the presence of the stimulus thereby obtaining a measured value; and

- Determining a difference between the reference value and the measured value,
  wherein UV/VIS-absorption of the sensor is used to determine the presence of an analyte or stimulus.

At first a sensor as described above is provided. The suitable embodiment of the sensor is selected according to the analytical problem to be solved, i.e. the stimulus applied to the sensor. The stimulus to be applied to the sensor may e.g. be pH, temperature, ionic strength or polarity of a solvent or a mixture. For detecting e.g. the presence of a solvent or determining the polarity of a solvent, it may e.g. be possible to select a sensor comprising a polymer brush formed from non-polar polymer molecules, e.g. polystyrene. The nanoparticles comprised in the sensor may be selected e.g. according to the physical parameter of the sensor used to detect the presence of stimulus, e.g. the presence of an analyte.

Then a suitable physical parameter of the sensor is selected to determine the presence of a stimulus. A suitable physical parameter may e.g. be an adsorption of light of various wavelength, electrical conductivity, magnetic properties, fluorescence/photoluminescence.

To obtain a reference value, the corresponding physical parameter of the sensor is detected without presence of the stimulus. A reference value may be obtained e.g. from a sensor in a dry state or from a sensor being in a defined state. E.g. for determining the polarity of a solvent, the reference value may be determined for a first solvent having a known polarity or may be detected in the absence of any analyte, i.e. in a dry state of the sensor. The determination of a reference value may be interpreted as calibration of the sensor.

The sensor is then contacted with the stimulus to be determined, e.g. a sample containing an analyte. The sample may be formed by a pure analyte or by a mixture of various compounds, one forming the analyte. Preferably the analyte is in liquid form. A liquid may interact with the polymer brush more easily than e.g. a gaseous probe. However, it is also possible to detect an analyte in a gaseous phase, e.g. a solvent vapor. The solvent vapor may e.g. effect a swelling of the polymer brush thereby generating a signal in the sensor.

After obtaining an equilibrium between sensor and applied stimulus, the physical parameter of the sensor is determined again thereby obtaining a measured value. The presence or intensity of the stimulus, e.g. an analyte or a feature of the analyte, may then be determined by comparing the reference value and the measured value.

In the present invention, UV/VIS-absorption of the sensor is used to determine the presence of an analyte or another stimulus.

The invention will now be described in more detail by way of examples and with reference to the accompanying figures.

Fig. 1      schematically shows the sensor employed in the method according to the invention and the states of the sensor in the presence of an analyte;

Fig. 2      shows a synthesis route for obtaining a sensor employed in the method according to the invention;

Fig. 3      shows an XPS carbon atom core level spectrum of bare polystyrene brushes;

Fig. 4      shows a TEM image of Au nanoparticles;

Fig. 5      shows (a) an IR spectrum and (b) an UV-VIS spectrum of 11-mercaptoundecanoic acid capped Au nanoparticles;

Fig. 6      shows topographic AFM images of polystyrene brushes (5 x 5 $\mu$m) (a) before and (b) after the immobilization of Au nanoparticles. Insert shows an enlarged view (1 x 1 $\mu$m) of a part of images;

Fig. 7      shows a XPS survey spectrum (take off angle 75°) of polystyrene brushes after the immobilization of Au nanoparticles;

Fig. 8      (a) shows UV-VIS spectra of Au-PS brushes in dry state and immersed in toluene (b) position of absorption maxima of Au-PS brushes in different surrounding media;

Fig. 9      shows a graph of the swelling kinetics of polystyrene brush in toluene, determined by ellipsometry; and

Fig. 10      shows a UV/VIS spectra of Au-polystyrene brushes in different surrounding media.

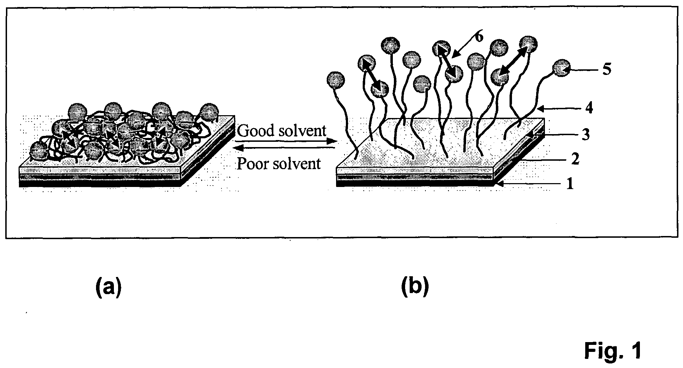

Fig. 1 schematically shows a sensor employed in the method according to the invention and the response of the sensor to an analyte. As an exemplary analyte are taken two solvents. A first solvent, designated as "good solvent" may intensely interact with the polymer brush of the sensor whereas a second solvent, designated as "poor solvent" will hardly interact with the polymer brush. Fig. 1(a) shows the sensor in a state in which the polymer brush is collapsed whereas in fig. 1(b) the polymer brush is in a swollen state.

The sensor comprises a substrate 1, e.g. a silicon wafer, that is covered with a thin adhesion enhancing layer 2 formed of e.g. silicon oxide. Adhesion enhancing layer 2 may comprise groups, e.g. hydroxyl groups, that may act to form a covalent bond. On top of adhesion enhancing layer 2 is deposited an anchoring layer 3. Anchoring layer comprises reactive groups, e.g. epoxy groups, that may form bonds towards corresponding groups in adhesion enhancing layer 2 or at polymer molecules 4. Polymer molecules 4 are tethered with one end to anchoring layer 3. Several of polymer molecules 4 form a polymer brush. To the free terminal end of polymer molecules 4 is bound a nanoparticle 5. The free ends of polymer molecules may float and therefore interparticle distance 6 of nanoparticles 5 may vary depending on the conformation of polymer molecules 4.

Fig. 1(b) shows the sensor in the presence of a good solvent, i.e. a solvent that will intensively interact with polymer molecules 4. The polymer brush formed from individual polymer molecules 4 therefore will swell with the polymer molecules 4 taking a expanded conformation. This allows the free terminal ends of polymer molecules 4 to float freely and interparticle distance 6 between two nanoparticles 5 will increase.

Fig. 1(a) shows the sensor in the presence of a poor solvent, i.e. a solvent that hardly interacts with the polymer molecules. Since the amount of solvent molecules present in the polymer brush is low due to their poor interaction with the polymer molecules, the polymer brush will shrink and collapse. Nanoparticles 5 bound to the free terminal ends of polymer molecules 4 therefore will come into closer relationship and interparticle distance will decrease.

If a physical parameter is taken for analysis that is sensitive to interparticle distance 6 of nanoparticles 5, a variation of this parameter can be triggered with a physical property of the analyte, i.e. the interaction of the analyte with polymer molecules 4.

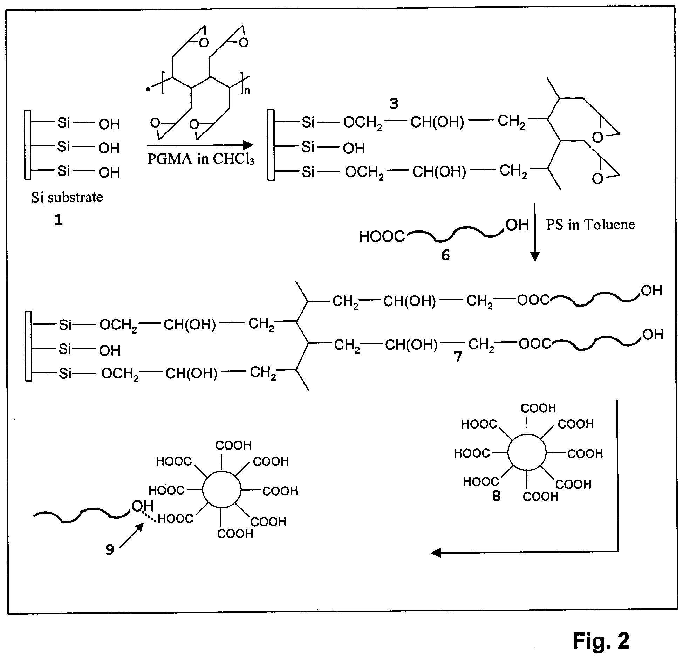

Fig. 2 shows schematically a synthesis route for preparation of a polymer brush and immobilization of nanoparticles for obtaining a sensor employed in the method according to the invention.

A silicon substrate 1 is provided which has been oxidized at the surface to obtain a thin oxide layer comprising surface bound hydroxyl groups. Before starting the synthesis of the sensor the wafer may be cleaned to remove impurities from the substrate surface. Onto the substrate surface is deposited a polymer comprising reactive groups to obtain an anchoring layer. As an exemplary polymer is used poly(glycidyl methacrylate) (PGMA) comprising epoxy groups as reactive groups. The PGMA may be provided in the form of a solution and may be applied to the substrate surface e.g. by spin coating. After removal of the solvent, the polymer layer is annealed such that hydroxyl groups provided on the substrate surface may react with some of the epoxy groups present in the PGMA to obtain an anchoring layer firmly bound to the silicon substrate by covalent bonds. In anchoring layer 3 are provided epoxy groups that have remained unreacted during annealing of the polymer layer. Substrate 1 with anchoring layer 3 bound to it is then reacted with a bifunctional polymer molecule 6. In fig 2 bifunctional polymer 6 carries a terminal carboxyl group and a terminal hydroxyl group. Other groups, however, may also be used. Bifunctional polymer molecule 6 may be provided in the form of a solution. The solvent is selected such that a sufficient concentration of the bifunctional polymer 6 may be obtained in the solution. The solution is applied to the substrate surface , e.g. by spin coating and the obtained layer is then annealed, e.g. by heating under reduced pressure. Terminal carboxyl group of bifunctional polymer 6 will react with reactive groups present in anchoring layer thereby forming a covalent bond. After annealing, bifunctional polymer 6 is bound with one end to substrate 1 leaving a free terminal end of the polymer carrying a terminal hydroxyl group. Unbound bifunctional polymer may be removed by rinsing or extraction with an appropriate solvent. A substrate is then obtained with a polymer brush 7 bound to its surface. For immobilization of nanoparticles, the polymer brush is reacted with surface functionalized nanoparticles 8. The nanoparticles carry carboxylic acid groups on their surface which may interact with terminal hydroxyl groups situated at the free end of polymer brush 7. Functionalized nanoparticles 8 are dispersed in a solvent, e.g. a solvent which will transfer polymer brush into a swollen state such that the free terminal ends of polymer brush 7 may float freely. The carboxylic acid groups of functionalized nanoparticle 8 may then form a hydrogen bond 9 with the terminal hydroxyl groups of polymer brush 7 to thereby immobilize the nanoparticles on the substrate surface. In a final step unbound functionalized nanoparticles 8 are removed from the substrate surface by several times rinsing. For rinsing, the same solvent may be used as taken for immobilization.

**Examples:**

Materials:

$\alpha$-hydroxy, $\omega$-carboxy terminated polystyrene ($M_n = 9500$ g mol$^{-1}$) was purchased from Polymer Source, Inc. and used as received. Polyglycidyl methacrylate (PGMA) ($M_n = 17.500$ g mol$^{-1}$) was synthesized by free radical polymerization of glycidyl methacrylate (Aldrich) as reported by Zdyrko et al., Langmuir 2003, 19, 10179 - 10178. Hydrogen tetrachloroaurate(III) (HAuCl$_4$ - · 4 H$_2$O), Tetraoctylammonium bromide (TOAB), 11-mercaptoundecanoic acid (MUA) and sodium borohydride all were purchased from Aldrich and used without additional purification. Highly polished single-crystal silicon wafers of {100} orientation with ca. 1.5 nm thick native silicon oxide layers were purchased from Semiconductor Processing Co. and used as substrates. Acetone, ethanol, dichloromethane (DCM), chloroform, cyclohexane, toluene and tetrahydrofuran (THF) were dried using standard methods before use.

Characterization:

Transmission electron microscopy (TEM) images of Au nanoparticles were recorded on Zeiss Omega 912 microscope at an accelerating voltage of 200 kV. Samples were prepared via drying a drop of toluene dispersion on a carbon coated copper grid. IR spectra of functionalized Au nanoparticles were recorded with Mattson Instruments Research Series 1 FTIR spectrometer. Prior to analysis, dried samples were mixed with KBr pellet. The thickness of polymer layers in the dry state was measured at $\lambda = 632.8$ nm and an angel of incidence of 70° with a null-ellipsometer in a polarizer compensator-sample analyzer (Multiscope, Optrel Berlin) as described by S. Minko et al. Langmuir 2002, 18, 289, L. Ionov et al. Macromol. Rapid Commun. 2004, 25, 360. From the obtained values, the distance between grafting points was calculated by the following equation:

$$d_g = \sqrt{\frac{M_n}{H \rho N_A}}$$

Where H is the ellipsometric thickness, $\rho$ is the mass density (1.04 g/cm$^3$), $N_A$ is Avogadro's number, and $M_n$ is the molecular weight. "In situ" ellipsometric measurements were performed to examine the swelling behavior of the polymer brush in different media. An ellipsometric cell with thin glass walls fixed at a known angle (68°) from a sample plane was used.

The measurements were performed for each sample after each step of the modification to use the measurements of the previous step as a reference for the simulation of ellipsometric data. The refractive indices used for the calculations were 3.858-i0.018, 1.4598, 1.525, 1.59 for silicon substrate, native silica layer, PGMA layer and PS brush. XPS experiments were performed with an AXISULTRA spectrometer (Kratos Analytical, U.K.) equipped with a monochromized Al K$\alpha$ X-ray source of 300 W at 20 mA. The survey spectra and high resolution spectra were obtained at analyzer's pass energy set value of 160 eV and 20 eV respectively. All spectra were charge compensated using the C$_x$H$_y$ component peak of the C 1s spectra at BE (binding energy) 285.00 eV as reference peak. To determine elemental ratios, normalized peak areas ($\Phi$) were calculated from peak areas (A) of survey spectra, respecting experimentally determined sensitivity factors and the spectrometer's transmission function using following equitation:

$$\Phi = \frac{A}{RSFTx}$$

Where RSF is respecting sensitivity factor and Tx is spectrometer's transmission function. The advancing water contact angle was measured using "DSA-10" Krüss equipment with an accuracy of 0.5°. UV-VIS absorption spectra were recorded with a Cary 50 spectrometer (Varian). Atomic force microscopy (AFM) studies were performed with a Dimension 3100 (Digital instruments, Inc., Santa Barbara, CA) microscope. Tapping mode was used to map the film morphology at ambient conditions. The surface coverage of adsorbed nanocrystals was estimated by the following equitation:

$$\varphi = \frac{100\% N \pi d^2}{4A}$$

Where d is the diameter of the nanocrystals and *N* is the number of nanocrystals detected per area A.

Preparation of polymer brushes

Polystyrene brushes were grown on silicon substrates by "grafting to" method as described by K. Swaminatha Iyer et al., Macro-molecules, 2003, 36, 6519 - 6526.

The wafers were first cleaned with dichloromethane 3 times in an ultrasonic bath for 10 min, then dried with nitrogen, and then dipped for 1.5 h in a $H_2O_2/NH_4OH$ (1:1) solution held at 80°C in a water bath. The wafers were then taken out from the $H_2O_2/NH_4OH$ solution, rinsed by stirring in millipore water 10 times for 5 min each, then dried with argon and stored over night under argon. The $SiO_2$ layer thickness was measured by ellipsometry and found to be around 1.5 nm.

To coat the wafers with a poly(glycidyl methacrylate) layer (PGMA), the wafers were spin-coated (speed=2000 rpm, accln=1000 rpm/s, time=20s) with a 0.02 weight % concentration solution of the polymer in chloroform followed by annealing for 10 min at 110°C in a vacuum oven. Then, the wafers were kept overnight in a vacuum oven at room temperature. The thickness of the obtained polymer layer was measured by ellipsometry and found to be around 2.0 nm. This PGMA layer works as an anchoring layer for brushes and substrate.

To prepare the polymer brushes, the wafers were spin-coated (speed=2000 rpm, accln=1000 rpm/s, time=20s) with a PS solution of 2 wt % concentration in dried toluene, and annealed in vacuum oven for 3 hours at 150°C. Then, soxhlet extraction was done for 4 hours in toluene to remove the physically absorbed polymer chains. After drying with argon, the thickness of the polymer layer was measured by ellipsometry and found to be around 6.0 nm.

Preparation of gold nanoparticles

Gold nanoparticles were synthesized by sodium borohydride ($NaBH_4$) reduction of chlorauric acid ($HAuCl_4$). The nanoparticle surface was modified with carboxyl groups using 11-mercaptoundecanoic acid (MUA), with the thiol group of this compound attached to the gold nanoparticle surface and the carboxyl groups left free at the surface.

328 mg of tetraoctylammonium bromide (TOAB) (0.6 mmol) were dissolved in 100 ml toluene and mixed with 50 ml of an aqueous solution of $HAuCl_4 \cdot 4H_2O$ (15 mM) at room temperature. Thereafter, a solution of 32.75 mg MUA (0.15 mmol) in 25 ml toluene was drop wise added into the reaction mixture under vigorous stirring. A freshly prepared 25 ml aqueous solution of 0.30 M $NaBH_4$ was added and the resulting mixture was alowed to stir for 1 h. The organic phase was separated, washed with distilled water and concentrated to dryness under reduced pressure. The black solid thus obtained was heat treated at 155 °C at a heating rate of 2 °C min$^{-1}$ for 30 min. The heat-treated product was dissolved in 10 ml of toluene, mixed with 200 ml of chloroform to remove excess TOAB and MUA, and then finally Au nanoparticles were separated through centrifugation.

Adsorption of nanoparticles on PS brushes

To immobilize gold nanoparticles on polymer brushes, nanoparticles were dispersed in toluene. Wafers with polymer brushes were dipped in dispersion of Au NPs overnight with stirring. Afterwards, non- or weakly adsorbed particles were removed by rinsing several times with toluene.

Results and discussion

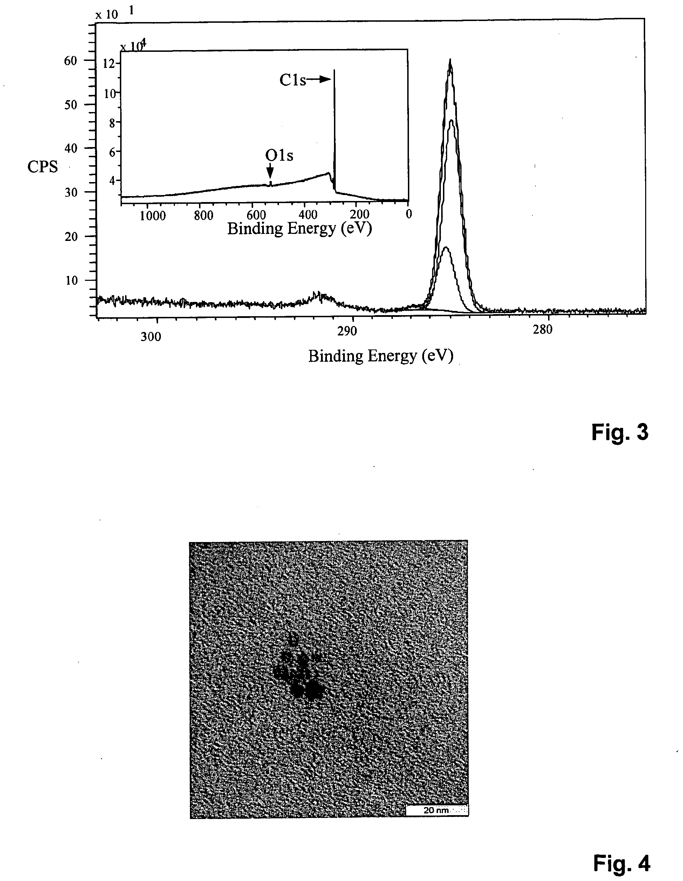

Well defined and homogenously distributed end functionalized PS brushes were prepared on silicon substrate exploiting well known "grafting to" approach. The process involves chemisorption of PGMA anchoring layer on silicon substrate followed by the grafting of bifunctionalized PS polymer chains. A thin layer of PGMA was deposited on cleaned silicon wafer by spin coating from 0.02 wt % solution in chloroform and annealed at 110°C for 10 minutes. It has been investigated earlier that PGMA can serve as a universal anchor for different types of polymer brushes on wide range of substrates including silicon, glass, alumina, gold, and silver by virtue of the high reactivity of its epoxy groups towards hydroxyl groups of these surfaces. The thickness of PGMA layer was found to be 2 $\pm$ 0.2 nm by ellipsometry. Bifunctionalized PS polymer chains were spin coated on PGMA layer and samples were annealed at 150 °C for 3 hrs. It leads to the grafting of PS chains on silicon substrate through the -COOH terminated ends and offers -OH terminated ends free for immobilization of Au nanoparticles, as confirmed by XPS. Fig. 3 shows a XPS carbon atom core level spectrum of bare polystyrene brushes, illustrating three deconvoluted peaks. The peak at 286.5 eV is characteristic of the carbon atom bonded with hydroxyl group. The insert in fig. 3 shows the wide scan XPS analysis of bare polystyrene brushes (take off angel: 75°). Chemical reaction between -COOH groups of PS chains and epoxy units located in the "loops" and "tails" sections of the attached PGMA macromolecules leads to the chemical attachment of PS chains on Si wafer. Ungrafted polymer was removed by Soxhlet extraction in toluene for 4 hrs and thickness of washed and dried PS brushes was measured as 6.0 nm by ellipsometry. Advancing contact angle of obtained PS brushes was found around 85° owing to the hydrophobic nature of polymer chains. Distance between the grafting points was estimated as 2.58 nm, which yielded the grafting density of obtained PS brushes as 0.15 nm$^{-2}$

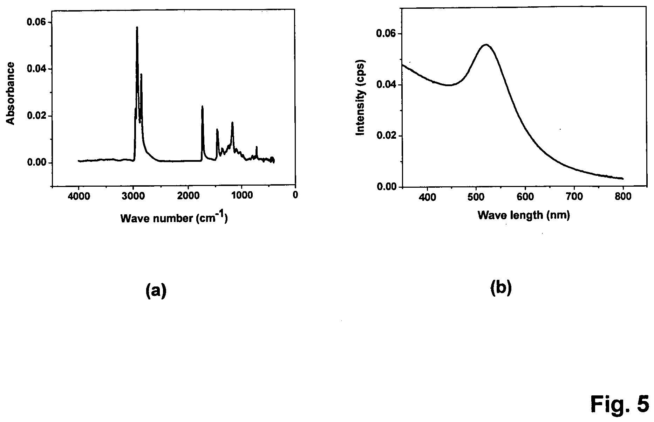

As shown in Figure 4, TEM image confirmed the formation of Au nanoparticles with a mean size of 5.2 nm. The surface of the Au nanoparticles was chemically protected with a polar and lipophilic group, 11-mercaptoundecanoic acid, exploiting the affinity of Au for the amines and thiol compounds. Presence of -COOH on the surface of nanoparticles was further confirmed by FTIR spectroscopy. Figure 5a shows a C=O stretching band at 1740 cm$^{-1}$ characteristic of free carboxylic acid groups. In addition, absence of S-H peak at 2550 cm$^{-1}$ confirms the attachment of this capping agent on the surface of gold nanoparticles through thiol function. Functionalization of Au nanoparticles with 11-mercaptoundecanoic acid not only provides -COOH moiety to interact with the -OH groups of polystyrene chains but also enables good solubility in toluene, which is a good solvent for polystyrene. Owing to the hydrophobic-hydrophobic interaction, all the polystyrene chains remain highly stretched in toluene. Simulation studies on polymer brushes have also revealed that chain ends are enriched in the top brush layer depending on chain stretching and grafting density. As a consequence of that, Au nanoparticles present in the solution have access to most of the end functionalities of highly stretched polymer chains and it facilitates the immobilization process. Figure 5b shows UV-VIS spectra of Au nanoparticles in toluene. A strong characteristic absorption peak at 520 nm can be observed.

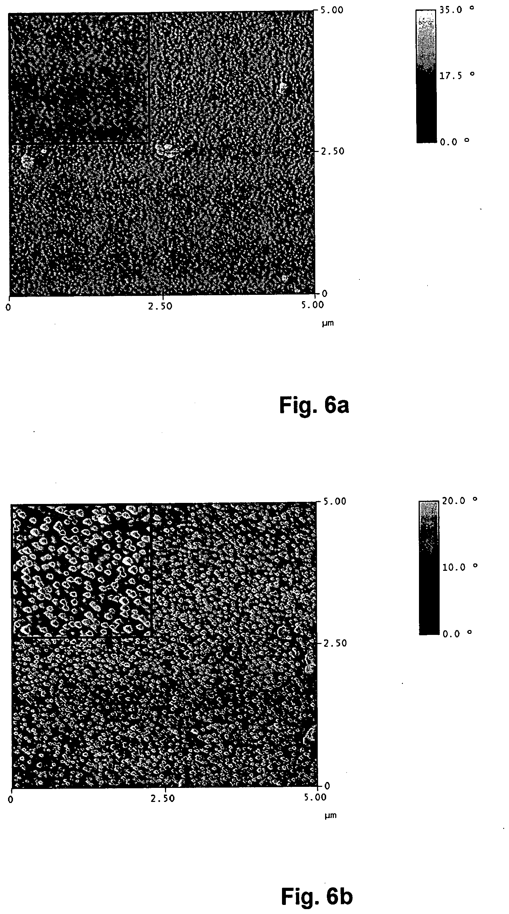

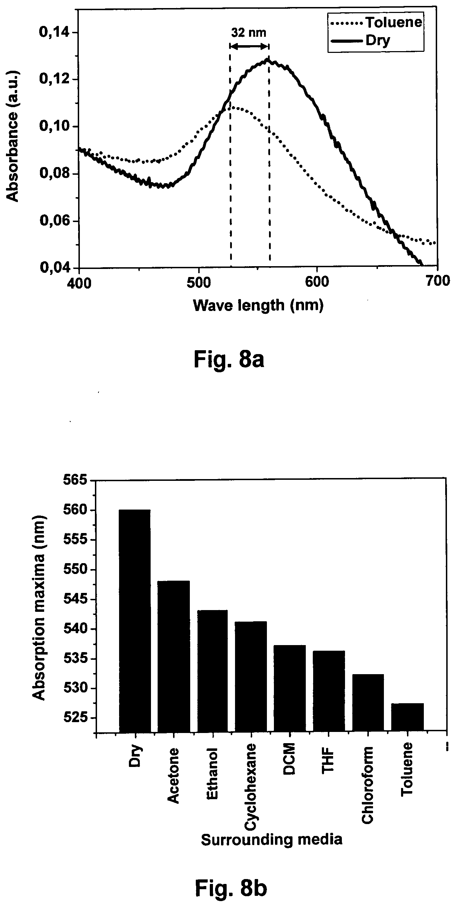

The immobilization of Au nanoparticles on polystyrene brushes was achieved by exploiting the physical interaction (hydrogen bonding) between end functionality of polystyrene chains (-OH groups) and surface functionality (-COOH groups) of Au nanoparticles. Overnight incubation of polystyrene brushes into the Au-toluene solution resulted in self assembly of Au nanoparticles on the polymer brushes. Figure 6 illustrates topographic AFM images of polymer brushes (a) before and (b) after the immobilization of Au nanoparticles. These results indicate that adsorbed Au nanoparticles formed a nearly uniform coverage on the polymer brushes. The root mean Square (rms) roughness of the bare polymer brushes was measured ≤ 0.5 nm, which increased significantly to the 3.3 nm after the absorption of Au nanoparticles. Moreover, a close inspection of these images reveals that sample has developed a "pebbled" appearance after the adsorption process, indicative of the presence of a dense layer of the nanoparticles on polystyrene brushes. AFM results indicate that Au nanoparticles cover approximately 15 % of brush surface area. It is necessary to mention that the immobilized Au nanoparticles appear to touch each other in the AFM images but this has to be attributed to AFM tip surface convolution effects. Preferably single Au nanoparticle adsorption was further confirmed by the absence of the optical signature of aggregate formation in the absorption spectrum of Au nanoparticles - an increase in the absorption at wavelengths greater than 600 nm (as shown in Figure 8a).

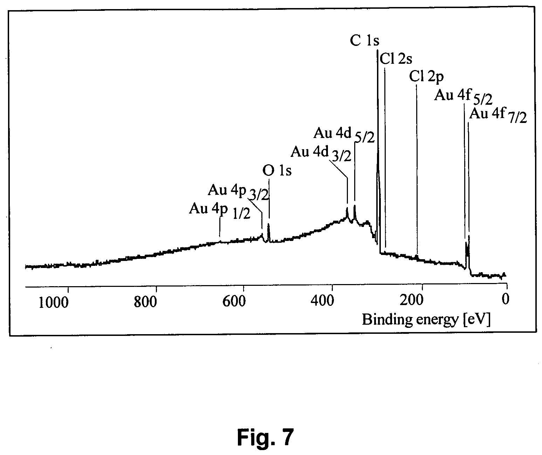

The presence of Au nanoparticles on polystyrene brushes was further proved by X-ray photoelectron spectroscopy (XPS) analysis. These measurements were carried out after vigorous washing and extraction of the samples. Figure 7 shows a XPS wide scan spectrum of the polystyrene brushes after the adsorption of Au nanoparticles (Au-PS brushes). In comparison to the wide scan spectrum of bare polystyrene brushes (shown in the insert of fig. 3), appearance of Au signals at the characteristic binding energies indicates presence of Au nanoparticles on polymer brushes. Moreover, in order to prove that adsorbed Au nanoparticles are located on the top of polymer brushes, XPS investigations of the samples were carried out at three different take-off angles (angle between surface normal and electron-optical axis of the spectrometer) namely 0°, 60° and 75° which are corresponding to the mean information depth of 8, 4 and 2 nm respectively. The XPS results indicate that the ratio of [Au]:[C] gradually increases from 0.009 to 0.016 with increasing take-off angle.

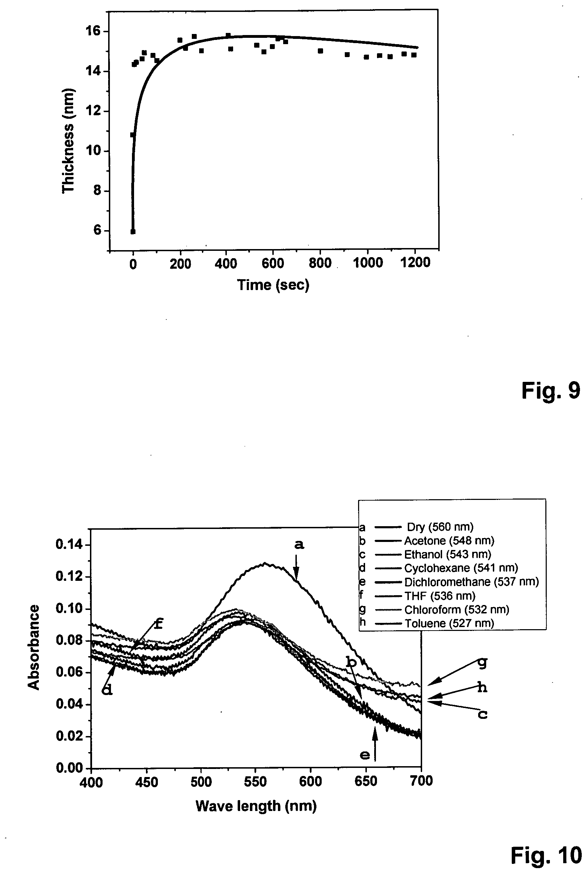

It has been realised that polymer chains, tethered to the substrates by one end in brush conformation, can show solvent induced swelling/deswelling behaviour. Two types of solvents have been proposed; poor solvents in which chains are always collapsed and good solvents that can induce strong stretching of the polymer chains with increased mobility of their free ends. The thickness of the swollen layers is governed by the balance between osmotic pressure and chain stretching. Various studies employing analytical theory simulation and neutron reflection have shown that stretched polymer chains in a polymer brush gradually shrink and finally collapse as the solvent quality decreases. The sensing material is designed to exploit this stimuli responsive property of polystyrene brushes. The proximity of the immobilized Au nanoparticles will be altered by swelling of their support polymer in response to the organic solvents. One can expect an increase in interparticle distance on swelling of the polymer brushes and a decrease in the same manner when brushes are collapsed. The change in proximity of immobilized Au nanoparticles could induce a shift in their plasmon absorption band. To observe the clear shift in surface plasmon resonance, the size and proximity of the Au nanoparticles immobilized on the polymer brushes are the critical factors. It is believed that the appropriate size of the Au nanoparticles for this purpose is <20 nm in diameter. Also, a narrow size distribution of the immobilized nanoparticles would be required for acquiring a sharp optical response. Therefore, the conditions for preparation of the Au nanoparticles were carefully determined and the concentration of Au nanoparticles in the absorption mixture. When samples were immersed into toluene which is considered as a good solvent for polystyrene, thickness of polymer brushes was found to increase from 6 nm to 14.7 nm by ellipsometry. Fig. 9 shows the swelling kinetics of polystyrene brushes in toluene as determined by ellipsometry. When this sample was removed from the toluene, dried and immersed into other solvents such as chloroform and ethanol, thickness had changed to 11.6 and 6.3 nm, respectively, suggesting the presence of a less stretched and collapsed conformation of polymer chains in these solvents. The degree of the swelling or conformation of polymer chains in solvents is expected to be governed by the quality of the solvent.

The UV-VIS measurements, conducted in various organic solvents elucidated the sensitivity and dynamic range of the optical response of nanohybrid assemblies. Figure 8a shows that the plasmon absorption band originally appeared with a maximum absorbance at 560 nm when brushes are in dry state. When polymer brushes were immersed in toluene, the absorption band was found to appear at 528 nm, showing the dramatic blue shift of 32 nm with respect to that of gold nanoparticles on dry polymer brushes. It can be attributed to the solvent induced swelling of polymer chains and higher mobility of their free ends in toluene, which caused increase in interparticle distance of Au nanoparticles, located at the top of polymer chains (as shown in fig. 1). Furthermore, when surrounding media was changed from toluene to ethanol, the absorption maxima was found to shift from 528 nm to 543 nm as ethanol is a poor solvent for polystyrene and brushes are expected to be collapsed in this surrounding medium. It is worth mentioning that this solvent induced shift in absorption maxima was found to be completely reversible. Variation in the position of plasmon absorption band with different organic solvents is depicted in Figure 8b. Corresponding UV-VIS spectra of Au-PS brushes in these solvents are shown in fig. 10. These results reveal that absorption band positions are controlled by the nature of the organic solvents.

Tokareva et al, *loc. cit.* demonstrated the shift in surface resonance band, when distance between the Au nanoparticles adsorbed on poly(2-vinylpyridine) polymer brushes and those on the substrate, was altered by pH induced swelling of P2VP chains. In comparison to this study, sensing mechanism of the nano-assemblies according to the invention is based on the change in interparticle distance of Au nanoparticles located on the top of polymer chains. Moreover, the employed polymer support i.e. polystyrene brushes are specifically responsive to a series of organic solvents as shown in Figure 8b. It is also important to emphasize here that this shift in absorption maxima is not induced by the change in refractive index of surrounding media. If it were so, it would have been observed only a red-shift in the maximum position with increase in refractive index of solvent. In contrast, it was observed that the absorption band position depends on the solvent quality irrespective of the refractive index. It strongly suggests that solvent induced change in conformation of chemically grafted polystyrene chains is the driving force for this modulation of the position of plasmon resonance band.

Conclusions:

In summary, it is demonstrated fabrication of an extremely straightforward but highly sensitive nanosensor, based on solvent induced swelling of polymer brushes coupled with surface plasmon resonance of Au nanoparticles. The described system has following advantages over previous studies (1) The change in plasmon absorption band of immobilized Au nanoparticles can be detected by a simple analytical tool i.e. UV-VIS absorption spectroscopy and the sensitivity is of same level as that measurable by more complex techniques (in terms of instruments and data processing), such as ellipsometry, SPR, or fluorescence labelling. (2) Particles have a good adhesion to the polymer support, which excludes the possibilities of their leakage after even multiple uses and (3) this approach is versatile in nature as it can be used for the fabrication of nanosensor devices based on temperature, pH and ionic strength responsive polymer brushes.

**Claims**

1.  Method for detecting an analyte or another stimulus comprising the steps:

    - providing a sensor comprising:

        - a substrate (1), wherein the substrate does not carry a gold layer;
        - a polymer brush (4) formed of polymer molecules anchored by a first end to a surface of the substrate and having a second, free end located on a terminal end opposite to the first end;
        - nanoparticles (5) fixed to the second end of the polymer brush,

    - selecting a physical parameter of the sensor;
    - determining the physical parameter thereby obtaining a reference value;
- applying a stimulus to the sensor;
- determining the physical parameter of the sensor in the presence of the stimulus, thereby obtaining a measured value;
- determining a difference between the reference value and the measured value,

wherein UV/VIS-absorption of the sensor is used to determine the presence of an analyte or another stimulus.

2. Method according to claim 1, wherein the polymer brush (4) comprises a bonding group at the second end.

3. Method according to one of the preceding claims, wherein the polymer brush (4) is formed from linear polymer chains.

4. Method according to one of the preceding claims, wherein the polymer brush (4) is formed from hydrocarbon repeating units.

5. Method according to one of the preceding claims, wherein the polymer brush (4) has a density of 0.05 to 0.5 polymer chains per nm$^2$.

6. Method according to one of the preceding claims, wherein the polymer brush (4) is bound to the substrate (1) via an anchoring layer (3).

7. Method according to claim 6, wherein the anchoring layer (3) is formed from a polymer comprising reactive groups.

8. Method according to one of the preceding claims, wherein the nanoparticles (5) are formed from metals.

9. Method according to one of the preceding claims, wherein the nanoparticles (5) are functionalized by surface molecules comprising a functional group that may bind to the bonding group of the polymer brush (4).

10. Method according to claim 9, wherein the surface molecule comprises a linking group for linking the surface molecule to the nanoparticle (5).

11. Method according to one of the preceding claims, wherein the nanoparticles have a size below 150 nm, preferably within a range of 1 to 50 nm.

12. Method according to one of the preceding claims, wherein the analyte is provided in liquid phase.

**Patentansprüche**

1. Verfahren zum Nachweisen eines Analyten oder eines anderen Stimulus, umfassend die Schritte:

   - Bereitstellen eines Sensors, umfassend:

      - ein Substrat (1), wobei das Substrat keine Goldschicht trägt;
      - eine Polymerbürste (4), gebildet aus Polymermolekülen, die durch ein erstes Ende an einer Oberfläche des Substrats verankert sind und die ein zweites, freies Ende aufweisen, das an einem terminalen Ende gegenüber zu dem ersten Ende lokalisiert ist;
      - Nanopartikel (5), die am zweiten Ende der Polymerbürste fixiert sind,

   - Auswählen eines physikalischen Parameters des Sensors;
   - Bestimmen des physikalischen Parameters, damit ein Referenzwert erhalten wird;
   - Aufbringen eines Stimulus auf den Sensor;
   - Bestimmen des physikalischen Parameters des Sensors in der Gegenwart des Stimulus, damit ein Messwert erhalten wird;
   - Bestimmen eines Unterschieds zwischen dem Referenzwert und dem Messwert,

   wobei UV/VIS-Absorption des Sensors zum Bestimmen der Gegenwart eines Analyten oder eines anderen Stimulus verwendet wird.

2. Verfahren gemäß Anspruch 1, wobei die Polymerbürste (4) einen bindenden Rest an dem zweiten Ende umfasst.

3. Verfahren gemäß einem der vorhergehenden Ansprüche, wobei die Polymerbürste (4) aus linearen Polymerketten gebildet ist.

4. Verfahren gemäß einem der vorhergehenden Ansprüche, wobei die Polymerbürste (4) aus Kohlenwasserstoff-Wiederholungseinheiten gebildet ist.

5. Verfahren gemäß einem der vorhergehenden Ansprüche, wobei die Polymerbürste (4) eine Dichte von 0,05 bis 0,5 Polymerketten pro nm$^2$ aufweist.

6. Verfahren gemäß einem der vorhergehenden Ansprüche, wobei die Polymerbürste (4) an das Substrat (1) über eine Ankerschicht (3) gebunden ist.

7. Verfahren gemäß Anspruch 6, wobei die Ankerschicht (3) aus einem reaktive Gruppen umfassenden Polymer gebildet ist.

8. Verfahren gemäß einem der vorhergehenden Ansprüche, wobei die Nanopartikel (5) aus Metallen gebildet sind.

9. Verfahren gemäß einem der vorhergehenden Ansprüche, wobei die Nanopartikel (5) durch Oberflächenmoleküle funktionalisiert sind, umfassend eine funktionelle Gruppe, die an den bindenden Rest der Polymerbürste (4) binden kann.

10. Verfahren gemäß Anspruch 9, wobei das Oberflächenmolekül einen bindenden Rest zum Binden des Oberflächenmoleküls an das Nanopartikel (5) umfasst.

11. Verfahren gemäß einem der vorhergehenden Ansprüche, wobei die Nanopartikel eine Größe unter 150 nm, bevorzugt in einem Bereich von 1 bis 50 nm aufweisen.

12. Verfahren gemäß einem der vorhergehenden Ansprüche, wobei der Analyt in flüssiger Phase bereitgestellt wird.

**Revendications**

1. Procédé de détection d'un analyte ou d'un autre stimulus comprenant les étapes :

   - la fourniture d'un capteur comprenant :

      ◦ un substrat (1) dans lequel le substrat ne porte pas une couche d'or ;
      ◦ une brosse polymère (4) formée de molécules polymère ancrées par une première extrémité à une surface du substrat et possédant une deuxième extrémité libre située sur une extrémité terminale opposée à la première extrémité ;
      ◦ des nanoparticules (5) fixées à la seconde extrémité de la brosse polymère,

   - le choix d'un paramètre physique du capteur ;
   - la détermination du paramètre physique, obtenant ainsi une valeur de référence;
   - l'application d'un stimulus sur le capteur ;
   - la détermination du paramètre physique du capteur en présence du stimulus, obtenant ainsi une valeur mesurée ;
   - la détermination d'une différence entre la valeur de référence et la valeur mesurée,

   dans lequel l'absorption UV/VIS du capteur est utilisée pour déterminer la présence d'un analyte ou d'un autre stimulus.

2. Procédé selon la revendication 1, dans lequel la brosse polymère (4) comprend un groupe de liaison à la deuxième extrémité.

3. Procédé selon l'une des revendications précédentes, dans lequel la brosse polymère (4) est formée de chaînes polymère linéaires.

4. Procédé selon l'une des revendications précédentes, dans lequel la brosse polymère (4) est formée d'unités de répétition d'hydrocarbure.

5. Procédé selon l'une des revendications précédentes, dans lequel la brosse polymère (4) possède une densité de 0,05 à 0,5 chaînes polymères par $nm^2$.

6. Procédé selon l'une des revendications précédentes, dans lequel la brosse polymère (4) est liée au substrat (1) par l'intermédiaire d'une couche d'ancrage (3).

7. Procédé selon la revendication 6, dans lequel la couche d'ancrage (3) est formée à partir d'un polymère comprenant des groupes réactifs.

8. Procédé selon l'une des revendications précédentes, dans lequel les nanoparticules (5) sont formées de métaux.

9. Procédé selon l'une des revendications précédentes, dans lequel les nanoparticules (5) sont fonctionnalisées par des molécules de surface comprenant un groupe fonctionnel qui peut se lier au groupe de liaison de la brosse polymère (4).

10. Procédé selon la revendication 9, dans lequel la molécule de surface comprend un groupe de liaison pour lier la molécule de surface à la nanoparticule (5).

11. Procédé selon l'une des revendications précédentes, dans lequel les nanoparticules ont une dimension inférieure à 150 nm, de préférence de l'ordre de 1 à 50 nm.

12. Procédé selon l'une des revendications précédentes, dans lequel l'analyte est fourni en phase liquide.

(a)　　　　　　　　(b)

**Fig. 1**

**Fig. 2**

**Fig. 3**

**Fig. 4**

(a)

(b)

**Fig. 5**

**Fig. 6a**

**Fig. 6b**

**Fig. 7**

**Fig. 8a**

**Fig. 8b**

**Fig. 9**

**Fig. 10**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 1215485 A1
- US 2005019849 A
- US 20060050268 A1
- US 20060286684 A1
- WO 03079402 A2

### Non-patent literature cited in the description

- **NATH et al.** *Anal. Chem.,* 2004, vol. 76, 5370-5378
- **MIRKIN.** *Nature,* 1996, vol. 382, 607
- **TOKAREVA et al.** *J. Am. Chem. Soc.,* 2004, vol. 126, 15950-15951
- **EMORY ; NIE.** *Science,* 1997, vol. 275, 1102-1106
- Investigations of Surfaces and Interfaces Part B. **BRANDT, E.S. ; COTTON, T.M.** Surface-enhanced Raman Scattering. John Wiley & Sons, 1993, vol. IXB, 633
- **MITSUISHI et al.** *Langmuir,* 2007, vol. 23 (14), 7472-7474
- **L. IONOV et al.** *Adv. Mater.,* 2006, vol. 18, 1453-1457
- **S. LIU et al.** *Phys. Chem. Chem. Phys. R. Soc. Chem.,* 2002, vol. 4 (24), 6059-6062
- **G. KALYUZHNY et al.** *Chemistry,* 2002, vol. 8 (17), 3849-3857
- **ZDYRKO et al.** *Langmuir,* 2003, vol. 19, 10179-10178
- **S. MINKO et al.** *Langmuir,* 2002, vol. 18, 289
- **L. IONOV et al.** *Macromol. Rapid Commun.,* 2004, vol. 25, 360
- **K. SWAMINATHA IYER et al.** *Macro-molecules,* 2003, vol. 36, 6519-6526